# EUROPÄISCHE PATENTSCHRIFT

(11) **EP 3 465 181 B1**
(45) Veröffentlichungstag und Bekanntmachung des Hinweises auf die Patenterteilung: **19.02.2020**
(21) Anmeldenummer: 17729796.7
(22) Anmeldetag: 24.05.2017
(51) Int. Cl.: G01N 23/20, G01N 33/20, G01N 23/20008, C21D 9/56, B21B 38/02

(54) **VORRICHTUNG UND VERFAHREN ZUM ERMITTELN EINER MIKROSTRUKTUR EINES METALLPRODUKTS SOWIE METALLURGISCHE ANLAGE**
DEVICE AND METHOD FOR DETERMINING THE MICROSTRUCTURE OF A METAL PRODUCT, AND METALLURGICAL INSTALLATION
DISPOSITIF ET PROCÉDÉ DE DÉTERMINATION D'UNE MICROSTRUCTURE D'UN PRODUIT MÉTALLIQUE ET INSTALLATION MÉTALLURGIQUE

(30) Priorität: 25.05.2016 DE 102016209181; 19.05.2017 DE 102017208576
(43) Veröffentlichungstag der Anmeldung: 10.04.2019
(73) Patentinhaber: SMS Group GmbH, 40237 Düsseldorf (DE)
(72) Erfinder: BIGLARI, Mostafa, 70173 Stuttgart (DE); SOMMERS, Ulrich, 40627 Düsseldorf (DE); KLINKENBERG, Christian, 58313 Herdecke (DE); RENARD, Michel, 4000 Liege (BE); RAYMOND, Guy, 4400 Ivoz-Ramet (BE); PENSIS, Oliver, 4420 Montegnee (BE); TERLAU, Tobias, 42489 Wülfrath (DE); KRAUTHÄUSER, Horst, 42579 Heiligenhau (DE)
(74) Vertreter: Klüppel, Walter
(86) Internationale Anmeldenummer: PCT/EP2017/062527
(87) Internationale Veröffentlichungsnummer: WO 2017/202904

(56) Entgegenhaltungen:
- EP-A1- 2 674 240
- DE-C- 556 383
- HERMANN-J. KOPINECK ET AL: "Industrial on-line texture determination in rolled steel strips", JOURNAL OF NONDESTRUCTIVE EVALUATION., Bd. 12, Nr. 1, 1. März 1993 (1993-03-01), Seiten 13-19, XP055285643, US ISSN: 0195-9298, DOI: 10.1007/BF00565904
- Timo Huttunen: "Ultra-thin X-ray window for detector applications", , 12. Dezember 2013 (2013-12-12), XP055398417, Gefunden im Internet: URL:http://www.esa-tec.eu/workspace/assets /files/tdo0066-52aaefcf77e9f.pdf [gefunden am 2017-08-14]
- R. M. FOWLER: "Some Recent Developments and Current Problems in Metal Analysis", ANALYTICAL CHEMISTRY, Bd. 31, Nr. 12, 1. Dezember 1959 (1959-12-01), Seiten 1949-1951, XP055398343, US ISSN: 0003-2700, DOI: 10.1021/ac60156a018
- O. Bruchwald ET AL: "Non-destructive in situ monitoring of the microstructural development in high performance steel components during heat treatment", La Metallurgia Italiana, 1. November 2015 (2015-11-01), XP055398350, Gefunden im Internet: URL:http://www.aimnet.it/allpdf/pdf_pubbli /nov15/Bruchwald.pdf [gefunden am 2017-08-11]

## Beschreibung

Die Erfindung betrifft eine Vorrichtung zum Ermitteln einer Mikrostruktur eines Metallprodukts während einer metallurgischen Herstellung des Metallprodukts nach Anspruch 1.

Des Weiteren betrifft die Erfindung eine metallurgische Anlage zum Herstellen eines Metallprodukts nach Anspruch 12.

Finale mechanische Eigenschaften eines metallurgisch hergestellten Metallprodukts gehören zu den wesentlichen Qualitätskriterien von solchen Metallprodukten. Die Mikrostruktur des Metallprodukts ist einer der bestimmenden Faktoren für diese mechanischen Eigenschaften.

Derzeit werden Metallprodukte durch strenges Befolgen eines bewährten Plans, der wichtige Werte für verschiedene gemessene Parameter beschreibt, metallurgisch hergestellt. Herkömmliche Messtechniken erfassen Parameter, wie beispielsweise eine Temperatur eines Metallprodukts, eine Bandgeschwindigkeit, eine Walzkraft und dergleichen, die in einem bestimmten Ausmaß in Beziehung mit der Mikrostruktur eines Metallprodukts gesetzt werden. Regelmäßige Prozessvariationen können die Beziehung zwischen den gemessenen Parametern und der Mikrostruktur eines Metallprodukts derart verändern, dass das Metallprodukt nicht mehr eine gewünschte Mikrostruktur und somit nicht die erforderlichen mechanischen Eigenschaften erhalten kann. Beispielsweise können während der Herstellung eines zweiphasigen Stahls relativ kleine Abweichungen von chemischen Zusammensetzungen das erforderliche Temperaturprofil verändern, dass erforderlich ist, um die gewünschten Anteile von Austenit und Ferrit während eines Glühens zu erhalten, was zu Metallprodukten führt, die zu hart und/oder zu spröde oder zu weich sind.

Der metallurgische Herstellungsprozess kann verbessert werden, wenn mehr Informationen über die Mikrostruktur und mechanische Eigenschaften eines Metallprodukts während dessen Herstellung verfügbar sind. Dies kann beispielsweise durch Verwendung von gegebenen messbaren Parametern, wie beispielsweise einer Temperatur eines Metallprodukts, eine chemische Zusammensetzung eines Metallprodukts, eine Walzkraft und dergleichen, erreicht werden, um einen oder mehrere Parameter zu berechnen, die in einer starken Beziehung mit der Mikrostruktur und/oder mechanischen Eigenschaften eines Metallprodukts stehen. Dies macht jedoch nachteiliger Weise ein Prozessmodell erforderlich, das umfangreiche Entwicklungsbemühungen erfordert.

Alternativ kann eine online-Messtechnik verwendet werden, mit der Parameter messbar sind, die in einer starken Beziehung mit der Mikrostruktur eines Metallprodukts stehen. Dies ist ebenfalls anspruchsvoll, da zerstörungsfreie, kontinuierliche, robuste und genaue Messungen in einer rauen Umgebung von Produktionsanlagen erforderlich sind.

Eine mögliche Technik zum Erfassen der Mikrostruktur eines Metallprodukts ist die Röntgenstrahlenbeugung. Bei dieser Technik wird das Metallprodukt mit Röntgenstrahlen bestrahlt, die gemäß der Bragg-Gleichung an den Gitterebenen der Kristallstruktur des Metallprodukts gebeugt werden. Ein Röntgendetektor erfasst die Intensität der gebeugten Röntgenstrahlen als Funktion des Beugungswinkels. Diese Daten können verwendet werden, um Parameter zu erhalten, welche die Mikrostruktur des bestrahlten Metallprodukts, wie beispielsweise dessen Kristallinität, Phasenzusammensetzung, Korngröße, innere und externe mechanische Spannungen und Gefüge, beschreiben. Jedoch ist diese online-Technik nicht weit verbreitet, da die dabei verwendeten Messgeräte durch Hitze oder Strahlung in der industriellen Anlagenumgebung beschädigt werden können.

Messanordnungen zum Messen von Eigenschaften eines Metallbands mittels Röntgenstrahlenbeugung sind beispielsweise aus JP 61 010 749 A, JP 03 817 812 B2 und JP 56 001 341 A, DE 38 25 830 C2, EP 0 352 423 B1 sowie Hermann-J. Kopineck et al: "Industrial on-line texture determination in rolled steel strips", J. Nondestructive Evaluation Bd.12 (1993), S.13-19 bekannt. Die Überprüfung eines Metallprodukts mit einer Schweißverbindung mittels Röntgenstrahlung ist beispielsweise aus EP 2 674 240 A1 bekannt. Röntgenstrahlungsdurchlässige Fenster sind beispielsweise aus Timo Huttunen: "Ultra-thin X-ray window for detector applications" (2013) bekannt.

Eine Aufgabe der Erfindung ist es, eine Ermittlung einer Mikrostruktur eines Metallprodukts während seiner metallurgischen Herstellung zuverlässig zu ermöglichen.

Diese Aufgabe wird durch die unabhängigen Patentansprüche gelöst. Vorteilhafte Ausgestaltungen sind in der nachfolgenden Beschreibung, den abhängigen Patentansprüchen und den Figuren wiedergegeben, wobei diese Ausgestaltungen jeweils für sich genommen oder in verschiedener Kombination von wenigstens zwei dieser Ausgestaltungen miteinander einen weiterbildenden, insbesondere auch bevorzugten oder vorteilhaften, Aspekt der Erfindung darstellen können. Ausgestaltungen der Vorrichtung und der metallurgischen Anlage können dabei Ausgestaltungen des Verfahrens entsprechen, und umgekehrt, selbst wenn im Folgenden hierauf im Einzelfall nicht explizit hingewiesen wird.

Eine erfindungsgemäße Vorrichtung zum Ermitteln einer Mikrostruktur eines Metallprodukts während einer metallurgischen Herstellung des Metallprodukts umfasst wenigstens eine Röntgenquelle, wenigstens einen Röntgendetektor, wenigstens eine, vorzugsweise geschlossene, Aufnahmekammer, innerhalb der die Röntgenquelle und/oder der Röntgendetektor angeordnet ist und die wenigstens ein für Röntgenstrahlung durchlässiges Fenster aufweist, und wenigstens eine Kühleinrichtung zum aktiven Kühlen der Aufnahmekammer.

Erfindungsgemäß werden die Aufnahmekammer und hierdurch die darin enthaltene Röntgenquelle bzw. der darin enthaltene Röntgendetektor aktiv gekühlt, was einen zuverlässigen Einsatz der erfindungsgemäßen Vorrichtung in einer Hochtemperaturumgebung, unter einer Schutzatmosphäre, unter einer Wärmeabstrahlung des Metallprodukts oder dergleichen möglich macht, ohne dass die Röntgenquelle bzw. der Röntgendetektor durch die Hitze beschädigt werden. Insbesondere wird hierdurch eine online-Ermittlung der Mikrostruktur eines Metallprodukts während seiner metallurgischen Herstellung, beispielsweise in einem Glühofen, möglich. Vorzugsweise wird die Aufnahmekammer derart aktiv gekühlt, dass eine Temperatur der Röntgenquelle bzw. des Röntgendetektors zumindest während der Messung mit der Vorrichtung unterhalb einer maximal zulässigen Betriebstemperatur der Röntgenquelle bzw. des Röntgendetektors liegt. Vorzugsweise wird die Aufnahmekammer derart aktiv gekühlt, dass die Temperatur der Aufnahmekammer und somit der darin angeordneten Röntgenquelle bzw. des darin angeordneten Röntgendetektors konstant gehalten wird, so dass die Messbedingungen konstant sind und Störungen und Abweichungen, die durch Temperaturschwankungen bedingt wären, nicht auftreten.

Durch diese online-Ermittlung der Mikrostruktur eines Metallprodukts kann eine Qualität des Metallprodukts schon bei dessen metallurgischer Herstellung sehr genau überwacht werden, wodurch ein gewünschter Qualitätsgrad des Metallprodukts sicher und reproduzierbar hergestellt werden kann. Hierdurch können Herstellungsausschüsse verringert und folglich Produktionskosten gesenkt werden. Zudem kann der Herstellungsprozess an die jeweilig online ermittelte Mikrostruktur des Metallprodukts angepasst werden, was die Ausbeute erhöht und Variationen der Qualität und Eigenschaften des Metallprodukts entlang der Produktlänge reduziert. Zudem können zu reparierende Metallprodukte und fehlerhafte Metallprodukte vor ihrer Weiterverarbeitung und einem Transport zu Kunden entfernt werden, was Verarbeitungskosten und Kundenforderungen verhindert. Ferner kann eine Kostenoptimierung bezüglich der Verarbeitung von Metallprodukten erfolgen, da die direkte online-Messung der Mikrostruktur eine Lockerung von herkömmlich erforderlichen Sicherheitsgrenzwerten erlaubt.

Die erfindungsgemäße Vorrichtung umfasst entweder eine aktiv gekühlte Aufnahmekammer zur Aufnahme der Röntgenquelle und eine separate aktiv gekühlte Aufnahmekammer zur Aufnahme des Röntgendetektors, oder alternativ sind sowohl die Röntgenquelle, als auch der Röntgendetektor in einer einzelnen aktiv gekühlten Aufnahmekammer angeordnet. Die erfindungsgemäße Vorrichtung kann derart ausgebildet sein, dass die Röntgenquelle und der Röntgendetektor auf derselben Seite oder auf einander gegenüberliegenden Seiten des Metallprodukts anordbar sind. Ist die Röntgenquelle in der Aufnahmekammer angeordnet, passiert die von der Röntgenquelle abgestrahlte Röntgenstrahlung das Fenster, trifft auf das Metallprodukt und gelangt von diesem zu dem Röntgendetektor (Reflexionsmessung). Ist der Röntgendetektor in einer separaten Aufnahmekammer mit einem Fenster angeordnet, gelangt die an dem Metallprodukt gebeugte Röntgenstrahlung durch dieses Fenster zu dem Röntgendetektor. Bei dieser Durchstrahlmessung wird das Metallprodukt in Dickenrichtung vollständig durchstrahlt. Ist der Röntgendetektor in derselben Aufnahmekammer wie die Röntgenquelle angeordnet, gelangt die an dem Metallprodukt gebeugte Röntgenstrahlung durch das Fenster der Aufnahmekammer zu dem Röntgendetektor.

Das Metallprodukt kann ein Metallband, eine Metallplatte, eine Bramme oder ein anderweitig langestreckt ausgebildetes Metallprodukt sein. Das Metallprodukt kann insbesondere ein Gießprodukt sein. Das Metallprodukt kann aus Aluminium, Eisen, Stahl, Nickel oder Kupfer hergestellt sein.

Die mit der erfindungsgemäßen Vorrichtung erfassten Daten können online und/oder offline verarbeitet werden. In letzterem Fall können die Daten zur Optimierung eines Prozessmodells oder dergleichen herangezogen werden. In ersterem Fall können die Daten durch eine Datenverarbeitung und/oder Algorithmen zum Steuern und/oder Regeln von Parametern einer zur Herstellung des Metallprodukts verwendeten metallurgischen Anlage verwendet werden. Beispielsweise können Walzkräfte, Temperaturen, Temperaturverhältnisse, Verarbeitungs- bzw. Produktgeschwindigkeiten und dergleichen in vorgelagerten Prozessschritten gesteuert und/oder geregelt werden. Diese Steuerung und/oder Regelung kann ein Bestandteil der Vorrichtung oder in ein externes System implementiert sein.

Erfindungsgemäß umfasst die Kühleinrichtung wenigstens eine Zuführeinrichtung zum Zuführen von wenigstens einem Kühlmedium zu der Aufnahmekammer, wobei die Aufnahmekammer kommunizierend mit der Zuführeinrichtung verbunden ist. Das Kühlmedium wird hierbei durch die Aufnahmekammer selbst geführt, um diese zu kühlen. Die Zuführeinrichtung kann einen offenen oder einen geschlossenen Kühlmediumkreislauf mit wenigstens einer Pumpe aufweisen.

Gemäß einer weiteren vorteilhaften Ausgestaltung umfasst die Kühleinrichtung wenigstens einen in der Aufnahmekammer angeordneten Wärmetauscher und wenigstens eine Zuführeinrichtung zum Zuführen von wenigstens einem Kühlmedium zu dem Wärmetauscher, wobei der Wärmetauscher kommunizierend mit der Zuführeinrichtung verbunden ist. Dadurch wird die Aufnahmekammer mittels des Wärmetauschers gekühlt. Durch die Wahl des jeweiligen Kühlmediums und durch eine Variation der Temperatur und/oder des Volumenstroms des Kühlmediums kann die Kühlwirkung der Kühleinrichtung an den jeweiligen Anwendungsfall angepasst werden. Die Zuführeinrichtung kann einen offenen oder einen geschlossenen Kühlmediumkreislauf mit wenigstens einer Pumpe aufweisen. Der Wärmetauscher kann beispielsweise als Kühlserpentine, Kühlspirale, Kühlwendel oder Flächenwärmetauscher ausgebildet sein.

Gemäß einer weiteren vorteilhaften Ausgestaltung umfasst die Kühleinrichtung wenigstens ein die Aufnahmekammer teilweise umschließendes Gehäuse mit wenigstens einer Öffnung, die mit dem Fenster verschlossen ist, und wenigstens eine Zuführeinrichtung zum Zuführen von wenigstens einem Kühlmedium zu dem Gehäuse, wobei das Gehäuse zumindest als Doppelwand mit beabstandet voneinander angeordneten Wänden ausgebildet ist und wenigstens ein Zwischenraum zwischen unmittelbar benachbart zueinander angeordneten Wänden kommunizierend mit der Zuführeinrichtung verbunden ist. Durch die Wahl des Kühlmediums sowie durch eine Variation von dessen Temperatur und Volumenstrom kann die Kühlwirkung der Kühleinrichtung an den jeweiligen Anwendungsfall angepasst werden. Die Zuführeinrichtung kann einen offenen oder einen geschlossenen Kühlmediumkreislauf mit wenigstens einer Pumpe aufweisen.

Gemäß einer weiteren vorteilhaften Ausgestaltung ist das Kühlmedium zumindest teilweise flüssig und/oder gasförmig. Die Wahl des Kühlmediums hängt von dem jeweiligen Anwendungsfall ab.

Erfindungsgemäß umfasst die Kühleinrichtung wenigstens eine Regeleinrichtung, die wenigstens einen Sensor und wenigstens eine mit dem Sensor verbundene Regelelektronik aufweist, wobei die Regelelektronik eingerichtet ist, die Zuführeinrichtung unter Berücksichtigung von Signalen des Sensors anzusteuern. Hierdurch ist es möglich, die Zuführeinrichtung und somit das Abführen der Wärmeenergie aus der Aufnahmekammer zu regeln, beispielsweise um die Aufnahmekammer auf eine konstante Temperatur zu regeln.

Gemäß einer weiteren vorteilhaften Ausgestaltung umfasst der Sensor wenigstens einen in der Aufnahmekammer angeordneten Temperatursensor und/oder wenigstens einen eine Temperatur des zugeführten Kühlmediums messenden Temperatursensor und/oder wenigstens einen eine Temperatur des abgeführten Kühlmediums messenden Temperatursensor und/oder wenigstens einen einen Volumendurchfluss des zugeführten Kühlmediums messenden Flusssensor und/oder wenigstens einen einen Druck des zugeführten Kühlmediums messenden Drucksensor und/oder wenigstens einen einen Volumendurchfluss des abgeführten Kühlmediums messenden Flusssensor und/oder wenigstens einen einen Druck des abgeführten Kühlmediums messenden Drucksensor.

Erfindungsgemäß ist die Regelelektronik eingerichtet, die Zuführeinrichtung derart anzusteuern, dass eine Temperatur einer Außenfläche und/oder einer Innenfläche des Gehäuses oberhalb einer Taupunkttemperatur einer Atmosphäre innerhalb und/oder außerhalb des Gehäuses liegt. Hierdurch wird die Kondensation von Wasser in der Nähe von der sensiblen Elektronik innerhalb der Aufnahmekammer beziehungsweise an der Außenfläche des Gehäuses vermieden.

Gemäß einer weiteren vorteilhaften Ausgestaltung umfasst die Vorrichtung wenigstens eine Einrichtung zum Trocknen und/oder Reinigen des zugeführten Kühlmediums. Hierdurch wird vermieden, dass Kondensat innerhalb der Aufnahmekammer beziehungsweise an dem Gehäuse entsteht. Das Kühlmedium wird liegt vorzugsweise gasförmig vor und wird mittels der Einrichtung getrocknet und/oder gereinigt, damit die Taupunkttemperatur des Kühlmediums unterhalb der Temperatur der Aufnahmekammer liegt beziehungsweise keine Unsauberkeiten in die Aufnahmekammer mitführt.

Erfindungsgemäß ist das Fenster derart ausgebildet, dass es von außerhalb der Aufnahmekammer auf das Fenster einfallende Infrarotstrahlung zumindest teilweise reflektiert oder absorbiert. Hierdurch wird teilweise oder vollständig verhindert, dass Infrarotstrahlung durch das Fenster zu der Röntgenquelle bzw. dem Röntgendetektor gelangt, was eine Erwärmung der Röntgenquelle bzw. des Röntgendetektors durch die Infrarotstrahlung verhindert. Vorzugsweise lässt das Fenster weniger als 50% der auf das Fenster einfallenden Infrarotstrahlung zu der Aufnahmekammer durch.

Gemäß einer weiteren vorteilhaften Ausgestaltung ist das Fenster aus einer Polyamid-Folie oder aus wenigstens eine Metallfolie oder aus Glas oder aus Keramik oder aus eine Kombination dieser Materialien gebildet. Dies verhindert, dass das Fenster der Röntgenstrahlung ein Beugungsmuster aufzwingt, was die Messung mit der Vorrichtung verfälschen könnte. Ein aus Keramik gebildetes Fenster kann die rauen Umgebung standhalten und dermaßen dünngestaltet werden, damit das Fenster so wenig wie möglich Röntgenstrahlung absorbiert oder beugt. Vorzugsweise enthält die Keramik vor allem leichte Elemente, die weniger mit der Röntgenstrahlung interferieren. Vorzugsweise ist das Fenster hitzebeständig ausgebildet.

Gemäß einer weiteren vorteilhaften Ausgestaltung ist das Fenster überwiegend aus leichten Elementen gebildet. Vorzugsweise besteht der Werkstoff des Fensters zu mehr als 50%, besonders bevorzugt zu mehr als 75%, insbesondere zu mehr als 90%, aus Elementen mit Atomnummer kleiner als 18. Gemäß einer weiteren vorteilhaften Ausgestaltung umfasst die Vorrichtung wenigstens eine mit der Röntgenquelle und/oder dem Röntgendetektor verbundene Bewegungseinrichtung mit der die Röntgenquelle und/oder der Röntgendetektor relativ zu dem Metallprodukt bewegbar sind. Hierdurch können die Röntgenquelle und der Röntgendetektor für eine Messung zu dem Metallprodukt bewegt und nach der Messung von dem Metallprodukt wegbewegt werden, um beispielsweise eine lokale Messung durchzuführen und/oder die Röntgenquelle und/oder den Röntgendetektor in gefährlichen Situationen in eine sichere Position zu bringen. Alternativ oder additiv können die Röntgenquelle und der Röntgendetektor während der Messung entlang eines Abschnitts des Metallprodukts bewegt werden. Alternativ oder additiv können die Röntgenquelle und der Röntgendetektor während einer Messung über einen Teil oder die gesamte Breite des Metallprodukts bewegt werden. Alternativ oder additiv kann das Metallprodukt gleichzeitig in seiner Längenrichtung an der Vorrichtung vorbeibewegt werden, um eine Messung auch über einen Längenabschnitt oder die gesamte Länge des Metallprodukts durchführen zu können. Alternativ oder additiv ist die Aufnahmekammer mit der Bewegungseinrichtung verbunden, um die Röntgendetektor und oder der Röntgenquelle zu bewegen.

Gemäß einer weiteren vorteilhaften Ausgestaltung umfasst die Vorrichtung wenigstens eine kommunizierend mit der Aufnahmekammer verbundene Spüleinrichtung, mit der die Aufnahmekammer mit wenigstens einem Spülmedium spülbar ist. Hierdurch kann beispielsweise eine Ablagerung von Staub oder anderen Verunreinigungen an der Röntgenquelle bzw. dem Röntgendetektor entfernt und/oder vermieden werden, welche die Funktionsfähigkeit der Vorrichtung beeinträchtigen könnten.

Gemäß einer weiteren vorteilhaften Ausgestaltung ist das Spülmedium zumindest teilweise flüssig und/oder gasförmig. Die Wahl des Kühlmediums hängt von dem jeweiligen Anwendungsfall ab. Vorzugsweise wird ein getrocknetes und/oder gereinigtes und/oder gekühltes Spülmedium eingesetzt.

Eine erfindungsgemäße metallurgische Anlage zum Herstellen eines Metallprodukts umfasst wenigstens eine Anlagenelektronik zum Steuern und/oder Regeln eines Betriebs von wenigstens einer Anlagenkomponente, mit der wenigstens ein metallurgischer Prozessschritt durchführbar ist, und wenigstens eine mit der Anlagenelektronik verbundene Vorrichtung nach einer der vorgenannten Ausgestaltungen oder einer beliebigen Kombination von wenigstens zwei dieser Ausgestaltungen miteinander, wobei die Anlagenelektronik eingerichtet ist, die Mikrostruktur des Metallprodukts aus Signalen der Vorrichtung zu ermitteln und die Anlagenkomponente unter Berücksichtigung der ermittelten Mikrostruktur zu steuern und/oder zu regeln.

Mit der metallurgischen Anlage sind die oben mit Bezug auf die Vorrichtung genannten Vorteile entsprechend verbunden. Die erfindungsgemäße metallurgische Anlage wird unter Berücksichtigung von online mittels der Vorrichtung erfassten Daten betrieben. Die Anlagenkomponente kann beispielsweise ein Walzwerk, ein Glühofen, eine Kühlstraße oder dergleichen sein.

Gemäß einem hier nicht beanspruchten Verfahren zum Ermitteln einer Mikrostruktur eines Metallprodukts während einer metallurgischen Herstellung des Metallprodukts unter Verwendung von wenigstens einer Röntgenquelle und wenigstens einem Röntgendetektor wird die Röntgenquelle und/oder der Röntgendetektor während der Ermittlung der Mikrostruktur des Metallprodukts aktiv gekühlt.

Mit dem Verfahren sind die oben mit Bezug auf die Vorrichtung genannten Vorteile entsprechend verbunden. Insbesondere kann die Vorrichtung gemäß einer der oben genannten Ausgestaltungen oder einer beliebigen Kombination von wenigstens zwei dieser Ausgestaltungen miteinander zur Durchführung des Verfahrens eingesetzt werden.

Im Folgenden wird die Erfindung unter Bezugnahme auf die anliegenden Figuren anhand bevorzugter Ausführungsformen beispielhaft erläutert, wobei die nachfolgend erläuterten Merkmale sowohl jeweils für sich genommen als auch in unterschiedlicher Kombination miteinander einen vorteilhaften oder weiterbildenden Aspekt der Erfindung darstellen können. Es zeigen:
- Figur 1:: eine schematische Darstellung eines Ausführungsbeispiels für eine erfindungsgemäße Vorrichtung; und
- Figur 2:: eine schematische Darstellung eines Ausführungsbeispiels für eine erfindungsgemäße metallurgische Anlage.

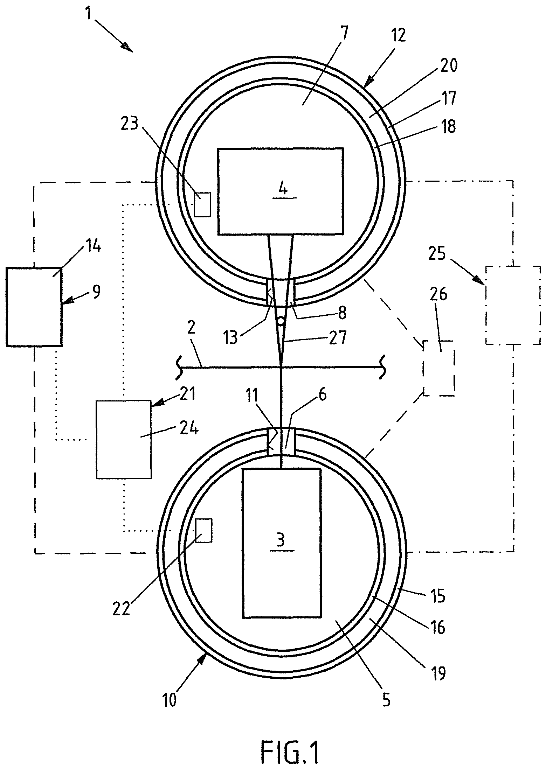

Figur 1 zeigt eine schematische Darstellung eines Ausführungsbeispiels für eine erfindungsgemäße Vorrichtung 1 zum Ermitteln einer Mikrostruktur eines Metallprodukts 2 während einer metallurgischen Herstellung des Metallprodukts 2.

Die Vorrichtung 1 umfasst eine auf einer Seite des Metallprodukts 2 angeordnete Röntgenquelle 3 und einen auf der gegenüberliegenden Seite des Metallprodukts 2 angeordneten Röntgendetektor 4. Des Weiteren umfasst die Vorrichtung 1 eine, vorzugsweise geschlossenen, Aufnahmekammer 5, innerhalb der die Röntgenquelle 3 angeordnet ist und die ein für Röntgenstrahlung durchlässiges, hitzebeständiges Fenster 6 aufweist. Zudem umfasst die Vorrichtung 1 eine weitere, vorzugsweise geschlossenen, Aufnahmekammer 7, innerhalb der der Röntgendetektor 4 angeordnet ist und die ein für Röntgenstrahlung durchlässiges, hitzebeständiges Fenster 8 aufweist. Jedes Fenster 6 bzw. 8 kann derart ausgebildet sein, dass es von außerhalb der jeweiligen Aufnahmekammer 5 bzw. 7 auf das Fenster 6 bzw. 8 einfallende Infrarotstrahlung teilweise oder vollständig reflektiert. Zudem kann jedes Fenster 6 bzw. 8 aus eine Polyamid-Folie oder aus wenigstens eine Metallfolie oder aus Glas oder aus Keramik oder dergleichen oder aus eine Kombination dieser Materialien gebildet sein. Vorzugsweise sind die Materialien amorph.

Die Vorrichtung 1 umfasst eine Kühleinrichtung 9 zum aktiven Kühlen der Aufnahmekammern 5 und 7. Die Kühleinrichtung 9 umfasst ein die Aufnahmekammer 5 teilweise umschließendes Gehäuse 10 mit wenigstens einer Öffnung 11, die mit dem Fenster 6 verschlossen ist. Zudem umfasst die Kühleinrichtung 9 ein die Aufnahmekammer 7 teilweise umschließendes Gehäuse 12 mit wenigstens einer Öffnung 13, die mit dem Fenster 8 verschlossen ist. Des Weiteren umfasst die Kühleinrichtung 9 eine Zuführeinrichtung 14 zum Zuführen eines Kühlmediums zu den Gehäusen 10 und 12. Jedes Gehäuse 10 bzw. 12 ist als Doppelwand mit beabstandet voneinander angeordneten Wänden 15 und 16 bzw. 17 und 18 ausgebildet ist. Ein Zwischenraum 19 bzw. 20 zwischen unmittelbar benachbart zueinander angeordneten Wänden 15 und 16 bzw. 17 und 18 ist kommunizierend mit der Zuführeinrichtung 14 verbunden. Das Kühlmedium ist zumindest teilweise flüssig oder gasförmig.

Die Kühleinrichtung 9 kann eine durch gestrichelte Linien schematisch angedeutete Regeleinrichtung 21 aufweisen, die einen in der Aufnahmekammer 5 angeordneten Sensor 22, einen in der Aufnahmekammer 7 angeordneten Sensor 23 und eine mit den Sensoren 22 und 23 verbundene Regelelektronik 24 aufweist, wobei die Regelelektronik 24 eingerichtet ist, die Zuführeinrichtung 14 unter Berücksichtigung von Signalen der Sensoren 22 und 23 anzusteuern. Die Sensoren umfassen wenigstens einen in der Aufnahmekammer angeordneten Temperatursensor oder einen Temperatursensor für das eingehendende Kühlmedium oder einen Temperatursensor für das ausgehendende Kühlmedium oder einen Flusssensor für das eingehende Kühlmedium oder einen Flusssensor für das ausgehende Kühlmedium oder eine Kombination dieser Sensoren. Die Regelelektronik 24 kann eingerichtet sein, die Zuführeinrichtung 14 derart anzusteuern, dass eine Temperatur einer Außenfläche und/oder einer Innenfläche des jeweiligen Gehäuses 10 bzw. 12 oberhalb einer Taupunkttemperatur einer Atmosphäre innerhalb und/oder außerhalb der Gehäuse 10 und 12 liegt.

Die Vorrichtung 1 kann eine mit der Röntgenquelle 3 und dem Röntgendetektor 4 mittelbar über das jeweilige Gehäuse 10 bzw. 12 verbundene, durch gestrichelte Linien stark schematisch angedeutete Bewegungseinrichtung 25 aufweisen, mit der die Röntgenquelle 3 und der Röntgendetektor 4 relativ zu dem Metallprodukt 2 bewegbar sind.

Ferner kann die Vorrichtung 1 eine kommunizierend mit den Aufnahmekammern 5 und 7 verbundene, durch gestrichelte Linien schematisch angedeutete Spüleinrichtung 26 aufweisen, mit der die Aufnahmekammern 5 und 7 mit einem Spülmedium spülbar sind, indem das Spülmedium durch die Aufnahmekammern 5 und 7 geblasen wird. Vorzugsweise ist die Spüleinrichtung 26 auf das jeweilige Fenster 6 bzw. gerichtet, um Unsauberkeiten, wie Staub und dergleichen, wegzublasen.

Zum Ermitteln der Mikrostruktur des Metallprodukts 2 strahlt die Röntgenquelle 3 Röntgenstrahlung 27 aus, die durch das Fenster 6 auf das Metallprodukt 2 fällt und von diesem gebeugt wird. Die gebeugte Röntgenstrahlung tritt durch das Fenster 8 in die Aufnahmekammer 7 ein und wird dort mit dem Röntgendetektor 4 detektiert.

Die Kühleinrichtung 9 kann alternativ oder additiv zwei Wärmetauscher aufweisen, die jeweils in einer Aufnahmekammer 5 bzw. 7 angeordnet sind. In einem solchen Fall kann die Zuführeinrichtung 14 zum Zuführen von wenigstens einem Kühlmedium zu den Wärmetauschern eingerichtet sein, wozu die Wärmetauscher kommunizierend mit der Zuführeinrichtung 14 verbunden sein können.

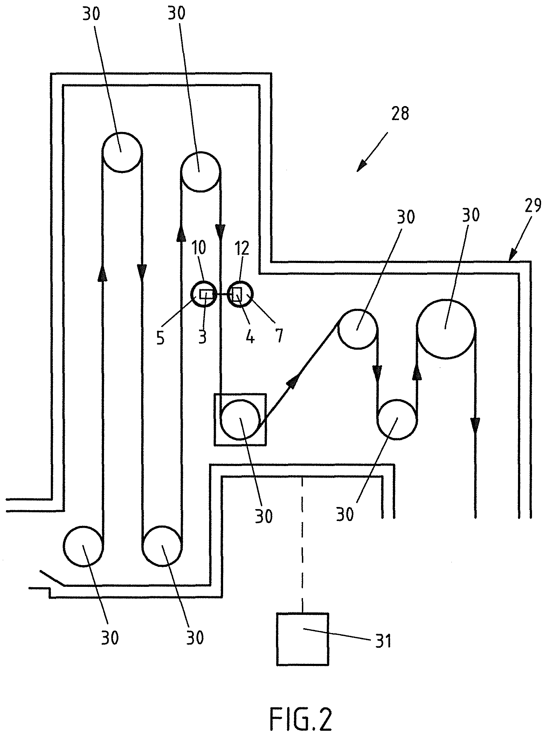

Figur 2 zeigt eine schematische Darstellung eines Ausführungsbeispiels für eine erfindungsgemäße metallurgische Anlage 28 zum Herstellen eines Metallprodukts 2 in Form eines Metallbands. Von der metallurgischen Anlage 28 ist ein Glühofen 29 gezeigt, durch den das über Umlenkrollen 30 geführte Metallprodukt 2 geführt wird. Innerhalb des Glühofens 29 herrschen hohe Temperaturen.

Die metallurgische Anlage 28 umfasst eine Anlagenelektronik 31 zum Steuern und/oder Regeln eines Betriebs von wenigstens einer Anlagenkomponente, insbesondere des Glühofens 29, mit der bzw. dem ein metallurgischer Prozessschritt durchführbar ist.

Ferner umfasst die metallurgische Anlage 28 eine mit der Anlagenelektronik 31 verbundene Vorrichtung 1 zum Ermitteln einer Mikrostruktur des Metallprodukts 2 während einer metallurgischen Herstellung des Metallprodukts 2, wobei die Vorrichtung 1 gemäß Figur 1 ausgebildet ist. Zur Vermeidung von Wiederholungen wird bezüglich der Vorrichtung 1 auf die obige Beschreibung zu Figur 1 verwiesen. Die Anlagenelektronik 31 ist eingerichtet, die Mikrostruktur des Metallprodukts 2 aus Signalen der Vorrichtung 1 zu ermitteln und die Anlagenkomponente, also den Glühofen 29, unter Berücksichtigung der ermittelten Mikrostruktur zu steuern und/oder zu regeln.

### Bezugszeichenliste

- 1: Vorrichtung
- 2: Metallprodukt
- 3: Röntgenquelle
- 4: Röntgendetektor
- 5: Aufnahmekammer
- 6: Fenster
- 7: Aufnahmekammer
- 8: Fenster
- 9: Kühleinrichtung
- 10: Gehäuse
- 11: Öffnung von 10
- 12: Gehäuse
- 13: Öffnung von 12
- 14: Zuführeinrichtung
- 15: Wand von 10
- 16: Wand von 10
- 17: Wand von 12
- 18: Wand von 12
- 19: Zwischenraum von 10
- 20: Zwischenraum von 12
- 21: Regeleinrichtung
- 22: Temperatursensor
- 23: Temperatursensor
- 24: Regelelektronik
- 25: Bewegungseinrichtung
- 26: Spüleinrichtung
- 27: Röntgenstrahlung
- 28: metallurgische Anlage
- 29: Glühofen
- 30: Umlenkrolle
- 31: Anlagenelektronik

## Patentansprüche

1. Vorrichtung (1) zum Ermitteln einer Mikrostruktur eines Metallprodukts (2) während einer metallurgischen Herstellung des Metallprodukts (2), aufweisend
- wenigstens eine Röntgenquelle (3),
- wenigstens einen Röntgendetektor (4),
- wenigstens eine Aufnahmekammer (5, 7), innerhalb der die Röntgenquelle (3) und/oder der Röntgendetektor (4) angeordnet ist und die wenigstens ein für Röntgenstrahlung durchlässiges Fenster (6, 8) aufweist, und
- wenigstens eine Kühleinrichtung (9) zum aktiven Kühlen der Aufnahmekammer (5, 7),
- wobei die Kühleinrichtung (9) wenigstens eine Zuführeinrichtung (14) zum Zuführen von wenigstens einem Kühlmedium zu der Aufnahmekammer (5, 7) aufweist,
- wobei die Aufnahmekammer (5, 7) kommunizierend mit der Zuführeinrichtung (14) verbunden ist,
**dadurch gekennzeichnet,**
- **dass** die Kühleinrichtung (9) wenigstens eine Regeleinrichtung (21) aufweist, die wenigstens einen Sensor (22, 23) und wenigstens eine mit dem Sensor (22, 23) verbundene Regelelektronik (24) aufweist, wobei die Regelelektronik (24) eingerichtet ist, die Zuführeinrichtung (14) unter Berücksichtigung von Signalen des Sensors (22, 23) anzusteuern,
- **dass** die Regelelektronik (24) eingerichtet ist, die Zuführeinrichtung (14) derart anzusteuern, dass eine Temperatur einer Außenfläche und/oder einer Innenfläche des Gehäuses (10, 12) oberhalb einer Taupunkttemperatur einer Atmosphäre innerhalb und/oder außerhalb des Gehäuses (10, 12) liegt, und
- **dass** das Fenster (6, 8) derart ausgebildet ist, dass es von außerhalb der Aufnahmekammer (5, 7) auf das Fenster (6, 8) einfallende Infrarotstrahlung zumindest teilweise reflektiert oder absorbiert.

2. Vorrichtung (1) nach Anspruch 1, **dadurch gekennzeichnet, dass** die Kühleinrichtung (9) wenigstens einen in der Aufnahmekammer (5, 7) angeordneten Wärmetauscher und wenigstens eine Zuführeinrichtung (14) zum Zuführen von wenigstens einem Kühlmedium zu dem Wärmetauscher aufweist, wobei der Wärmetauscher kommunizierend mit der Zuführeinrichtung (14) verbunden ist.

3. Vorrichtung (1) nach Anspruch 1 oder 2, **dadurch gekennzeichnet, dass** die Kühleinrichtung (9) wenigstens ein die Aufnahmekammer (5, 7) teilweise umschließendes Gehäuse (10, 12) mit wenigstens einer Öffnung (11, 13), die mit dem Fenster (6, 8) verschlossen ist, und wenigstens eine Zuführeinrichtung (14) zum Zuführen von wenigstens einem Kühlmedium zu dem Gehäuse (10, 12) aufweist, wobei das Gehäuse (10, 12) zumindest als Doppelwand mit beabstandet voneinander angeordneten Wänden (15, 16, 17, 18) ausgebildet ist und wenigstens ein Zwischenraum (19, 20) zwischen unmittelbar benachbart zueinander angeordneten Wänden (15, 16, 17, 18) kommunizierend mit der Zuführeinrichtung (14) verbunden ist.

4. Vorrichtung (1) nach einem der Ansprüche 1 bis 3, **dadurch gekennzeichnet, dass** das Kühlmedium zumindest teilweise flüssig und/oder gasförmig ist.

5. Vorrichtung (1) nach einem der Ansprüche 1 bis 4, **dadurch gekennzeichnet, dass** der Sensor (22, 23) wenigstens einen in der Aufnahmekammer (5, 7) angeordneten Temperatursensor und/oder wenigstens einen eine Temperatur des zugeführten Kühlmediums messenden Temperatursensor und/oder wenigstens einen eine Temperatur des abgeführten Kühlmediums messenden Temperatursensor und/oder wenigstens einen einen Volumendurchfluss des zugeführten Kühlmediums messenden Flusssensor und/oder wenigstens einen einen Druck des zugeführten Kühlmediums messenden Drucksensor und/oder wenigstens einen einen Volumendurchfluss des abgeführten Kühlmediums messenden Flusssensor und/oder wenigstens einen einen Druck des abgeführten Kühlmediums messenden Drucksensor aufweist.

6. Vorrichtung (1) nach einem der Ansprüche 1 bis 5, **gekennzeichnet durch** wenigstens eine Einrichtung zum Trocknen und/oder Reinigen des zugeführten Kühlmediums.

7. Vorrichtung (1) nach einem der Ansprüche 1 bis 6, **dadurch gekennzeichnet, dass** das Fenster (6, 8) aus einer Polyamid-Folie oder aus wenigstens eine Metallfolie oder aus Glas oder aus Keramik oder aus eine Kombination dieser Materialien gebildet ist.

8. Vorrichtung (1) nach einem der Ansprüche 1 bis 7, **dadurch gekennzeichnet, dass** das Fenster (6, 8) aus überwiegend aus leichten Elementen gebildet ist.

9. Vorrichtung (1) nach einem der Ansprüche 1 bis 8, **gekennzeichnet durch** wenigstens eine mit der Röntgenquelle (3) und/oder dem Röntgendetektor (4) verbundene Bewegungseinrichtung (25) mit der die Röntgenquelle (3) und/oder der Röntgendetektor (4) relativ zu dem Metallprodukt (2) bewegbar sind.

10. Vorrichtung (1) nach einem der Ansprüche 1 bis 9, **gekennzeichnet durch** wenigstens eine kommunizierend mit der Aufnahmekammer (5, 7) verbundene Spüleinrichtung (26), mit der die Aufnahmekammer (5, 7) mit wenigstens einem Spülmedium spülbar ist.

11. Vorrichtung (1) nach Anspruch 10, **dadurch gekennzeichnet, dass** das Spülmedium zumindest teilweise flüssig und/oder gasförmig ist.

12. Metallurgische Anlage (28) zum Herstellen eines Metallprodukts (2), aufweisend wenigstens eine Anlagenelektronik (31) zum Steuern und/oder Regeln eines Betriebs von wenigstens einer Anlagenkomponente (29), mit der wenigstens ein metallurgischer Prozessschritt durchführbar ist, **gekennzeichnet durch** wenigstens eine mit der Anlagenelektronik (31) verbundene Vorrichtung (1) nach einem der Ansprüche 1 bis 11, wobei die Anlagenelektronik (31) eingerichtet ist, die Mikrostruktur des Metallprodukts (2) aus Signalen der Vorrichtung (1) zu ermitteln und die Anlagenkomponente (29) unter Berücksichtigung der ermittelten Mikrostruktur zu steuern und/oder zu regeln.

## Claims

1. Device (1) for determining a microstructure of a metal product (2) during metallurgical production of the metal product (2), comprising
- at least one X-ray source (3),
- at least one X-ray detector (4),
- at least one receiving chamber (5, 7), within which the X-ray source (3) and/or the X-ray detector (4) is or are arranged and which has at least one window (6, 8) permeable by X-ray radiation, and
- at least one cooling device (9) for active cooling of the receiving chamber (5, 7),
- wherein the cooling device (9) has at least one feed device (14) for feeding at least one cooling medium to the receiving chamber (5, 7) and
- wherein the receiving chamber (5, 7) has communicating connection with the feed device (14),
**characterised in that**
- the cooling device (9) comprises at least one regulating device (21) comprising at least one sensor (22, 23) and at least one electronic regulating system (24) connected with the sensor (22, 23), wherein the electronic regulating system (24) is arranged to activate the feed device (14) taking into account signals of the sensor (22, 23),
- the electronic regulating system (24) is arranged to activate the feed device (14) in such a way that a temperature of an outer surface and/or an inner surface of the housing (10, 12) lies above a dew point temperature of an atmosphere inside and/or outside the housing (10, 12) and
- the window (6, 8) is constructed in such a way that it at least partly reflects or absorbs infrared radiation incident on the window (6, 8) from outside the receiving chamber (5, 7).

2. Device (1) according to claim 1, **characterised in that** the cooling device (9) comprises at least one heat exchanger arranged in the receiving chamber (5, 7) and at least one feed device (14) for feeding at least one cooling medium to the heat exchanger, wherein the heat exchanger is in communicating connection with the feed device (14).

3. Device (1) according to claim 1 or 2, **characterised in that** the cooling device (9) comprises at least one housing (10, 12), which partly encloses the receiving chamber (5, 7), with at least one opening (11, 13), which is closed by the window (6, 8), and at least one feed device (14) for feeding at least one cooling medium to the housing (10, 12), wherein the housing (10, 12) is constructed at least as a double wall with walls (15, 16, 17, 18) arranged at a spacing from one another, and at least one intermediate space (19, 20) between walls (15, 16, 17, 18) arranged directly adjacent to one another is in communicating connection with the feed device (14).

4. Device (1) according to any one of claims 1 to 3, **characterised in that** the cooling medium is at least in part liquid and/or gaseous.

5. Device (1) according to any one of claims 1 to 4, **characterised in that** the sensor (22, 23) comprises at least one temperature sensor arranged in the receiving chamber (5, 7) and/or at least one temperature sensor measuring a temperature of the fed cooling medium and/or at least one temperature sensor measuring a temperature of the discharged cooling medium and/or at least one flow sensor measuring a volume throughflow of the fed cooling medium and/or at least one pressure sensor measuring a pressure of the fed cooling medium and/or at least one flow sensor measuring a volume throughflow of the discharged cooling medium and/or at least one pressure sensor measuring a pressure of the discharged cooling medium.

6. Device (1) according to any one of claims 1 to 5, **characterised by** at least one device for drying and/or cleaning the fed cooling medium.

7. Device (1) according to any one of claims 1 to 6, **characterised in that** the window (6, 8) is formed from a polyamide foil or from at least one metal foil or from glass or from ceramic or from a combination of these materials.

8. Device (1) according to any one of claims 1 to 7, **characterised in that** the window (6, 8) is formed predominantly from light elements.

9. Device (1) according to any one of claims 1 to 8, **characterised by** at least one movement device (25), which is connected with the X-ray source (3) and/or the X-ray detector (4) and by which the X-ray source (3) and/or the X-ray detector (4) is or are movable relative to the metal product (2).

10. Device (1) according to any one of claims 1 to 9, **characterised by** at least one flushing device (26), which is in communicating connection with the receiving chamber (5, 7) and by which the receiving chamber (5, 7) can be flushed with at least one flushing medium.

11. Device (1) according to claim 10, **characterised in that** the flushing medium is at least in part liquid and/or gaseous.

12. Metallurgical plant (28) for producing a metal product (2), comprising at least one plant electronic system (31) for controlling and/or regulating an operation of at least one plant component (29) by which at least one metallurgical process step can be carried out, **characterised by** at least one device (1), which is connected with the plant electronic system (31), according to any one of claims 1 to 11, wherein the plant electronic system (31) is arranged to determine to the microstructure of the metal product (2) from signals of the device (1) and to control and/or regulate the plant component (29) taking into account the determined microstructure.

## Revendications

1. Dispositif (1) destiné à déterminer une microstructure d'un produit métallique (2) au cours d'une fabrication métallurgique du produit métallique (2), présentant :
- au moins une source de rayons X (3) ;
- au moins un détecteur de rayons X (4) ;
- au moins une chambre de réception (5, 7) à l'intérieur de laquelle est/sont disposé(s) la source de rayons X (3) et/ou le détecteur de rayons X (4) et qui présente au moins une fenêtre (6, 8) perméable aux rayons X ; et
- au moins un mécanisme de refroidissement (9) pour le refroidissement actif de la chambre de réception (5, 7) ;
- dans lequel le mécanisme de refroidissement (9) présente au moins un mécanisme d'alimentation (14) pour l'alimentation de la chambre de réception (5, 7) avec au moins un milieu de refroidissement ;
- dans lequel la chambre de réception (5, 7) est reliée en communication avec le mécanisme d'alimentation (14) ;
**caractérisé**
- **en ce que** le mécanisme de refroidissement (9) présente au moins un mécanisme de réglage (21) qui présente au moins un capteur (22, 23) et au moins une électronique de réglage (24) reliée au capteur (22, 23) ; dans lequel l'électronique de réglage (24) est conçue pour la commande du mécanisme d'alimentation (14) en prenant en compte des signaux du capteur (22, 23) ;
- **en ce que** l'électronique de réglage (24) est conçue pour la commande du mécanisme d'alimentation (14) d'une manière telle qu'une température d'une surface externe et/ou d'une surface interne du boîtier (10, 12) est supérieure à une température de point de rosée d'une atmosphère régnant à l'intérieur et/ou à l'extérieur du boîtier (10, 12) ; et
- **en ce que** la fenêtre (6, 8) est réalisée d'une manière telle qu'elle réfléchit ou qu'elle absorbe au moins en partie un rayonnement infrarouge qui heurte la fenêtre (6, 8) à partir de l'extérieur de la chambre de réception (5, 7).

2. Dispositif (1) selon la revendication 1, **caractérisé en ce que** le mécanisme de refroidissement (9) présente au moins un échangeur de chaleur disposé dans la chambre de réception (5, 7) et au moins un mécanisme d'alimentation (14) pour l'alimentation de l'échangeur de chaleur avec au moins un milieu de refroidissement ; dans lequel l'échangeur de chaleur est relié en communication avec le mécanisme d'alimentation (14).

3. Dispositif (1) selon la revendication 1 ou 2, **caractérisé en ce que** le mécanisme de refroidissement (9) présente au moins un boîtier (10, 12) entourant en partie la chambre de réception (5, 7), comprenant au moins une ouverture (11, 13) qui est fermée avec la fenêtre (6, 8), et au moins un mécanisme d'alimentation (14) pour l'alimentation du boîtier (10, 12) avec au moins un milieu de refroidissement ; dans lequel le boîtier (10, 12) est réalisé au moins sous la forme d'une double paroi comprenant des parois (15, 16 ,17, 18) disposées à distance les unes des autres, et au moins un espace intermédiaire (19, 20) entre des parois (15, 16, 17, 18) disposées en position directement voisines les unes des autres est relié en communication avec le mécanisme d'alimentation (14).

4. Dispositif (1) selon l'une quelconque des revendications 1 à 3, **caractérisé en ce que** le milieu de refroidissement est au moins en partie liquide et/ou gazeux.

5. Dispositif (1) selon l'une quelconque des revendications 1 à 4, **caractérisé en ce que** le capteur (22, 23) présente au moins un capteur de la température disposé dans la chambre de réception (5, 7) et/ou au moins un capteur de la température qui mesure une température du milieu de refroidissement alimenté et/ou au moins un capteur de la température qui mesure une température du milieu de refroidissement évacué et/ou au moins un capteur du flux qui mesure un écoulement volumique du milieu de refroidissement alimenté et/ou au moins un capteur de la pression qui mesure une pression du milieu de refroidissement alimenté et/ou au moins un capteur du flux qui mesure un écoulement volumique du milieu de refroidissement évacué et/ou au moins un capteur de la pression qui mesure une pression du milieu de refroidissement évacué.

6. Dispositif (1) selon l'une quelconque des revendications 1 à 5, **caractérisé par** au moins un mécanisme destiné au séchage et/ou à la purification du milieu de refroidissement alimenté.

7. Dispositif (1) selon l'une quelconque des revendications 1 à 6, **caractérisé en ce que** la fenêtre (6, 8) est réalisée à partir d'une feuille de polyamide ou à partir d'au moins une feuille de métal ou en verre ou en céramique ou encore à partir d'une combinaison de ces matières.

8. Dispositif (1) selon l'une quelconque des revendications 1 à 7, **caractérisé en ce que** la fenêtre (6, 8) est réalisée à titre principal à partir d'éléments légers.

9. Dispositif (1) selon l'une quelconque des revendications 1 à 8, **caractérisé par** au moins un mécanisme de déplacement (25) relié à la source de rayons X (3) et/ou au détecteur de rayons X (4), avec lequel on peut déplacer la source de rayons X (3) et/ou le détecteur de rayons X (4) par rapport au produit métallique (2).

10. Dispositif (1) selon l'une quelconque des revendications 1 à 9, **caractérisé par** au moins un mécanisme de rinçage (26) relié en communication avec la chambre de réception (5, 7), avec lequel la chambre de réception (5, 7) peut être rincée avec au moins un milieu de rinçage.

11. Dispositif (1) selon la revendication 10, **caractérisé en ce que** le milieu de rinçage est au moins en partie liquide et/ou gazeux.

12. Installation métallurgique (28) pour la fabrication d'un produit métallique (2), présentant au moins une électronique d'installation (31) pour la commande et/ou pour le réglage d'un fonctionnement d'au moins un composant (29) de l'installation, avec lequel on peut mettre en oeuvre au moins une étape d'un processus métallurgique, **caractérisé par** au moins un dispositif (1) relié à l'électronique (31) de l'installation, selon l'une quelconque des revendications 1 à 11 ; dans laquelle l'électronique (31) de l'installation est conçue pour déterminer la microstructure du produit métallique (2) à partir de signaux du dispositif (1) et pour commander et/ou pour régler le composant (29) de l'installation en prenant en compte la microstructure déterminée.
